# EUROPEAN PATENT SPECIFICATION

(11) **EP 1 623 722 B1**
(45) Date of publication and mention of the grant of the patent: **23.06.2010**
(21) Application number: 04730634.5
(22) Date of filing: 30.04.2004
(51) Int. Cl.: A61K 47/38, A61K 9/08, A61P 27/04

(54) **COMPOSITION FOR OPHTHALMIC USE**
ZUSAMMENSETZUNG ZUR OPHTHALMISCHEN VERWENDUNG
COMPOSITION OPHTALMIQUE

(30) Priority: 15.05.2003 JP 2003137828
(43) Date of publication of application: 08.02.2006
(73) Proprietor: Menicon Co., Ltd., Nagoya-shi, Aichi 460-0006 (JP)
(72) Inventor: TSUZUKI, Akira, c/o MENICON CO., LTD., Kasugai-shi, Aichi 487-0032 (JP); IWAMURO, Sayuri, c/o MENICON CO., LTD., Kasugai-shi, Aichi 487-0032 (JP); TANIKAWA, Sadayasu, c/o MENICON CO., LTD., Kasugai-shi, Aichi 487-0032 (JP)
(74) Representative: Paget, Hugh Charles Edward
(86) International application number: PCT/JP2004/005837
(87) International publication number: WO 2004/100993

(56) References cited:
- WO-A1-99/16447
- WO-A1-02/055118
- JP-A- 2001 318 348
- JP-A- 2002 322 048
- JP-A- 2003 055 262
- JP-A- 2003 183 157
- US-A- 5 599 534

## Description

### TECHNICAL FIELD

The present invention relates in general to the use of an ophthalmic solution as eye drops, and which provides an enhanced comfort.

### BACKGROUND ART

In these days, contact lens wearers are increased, and as the contact lens wearer increases, there are many contact lens wearers, who complain of symptoms, such as dry feeling (dryness of the eye), eye strain, dimness of sight, and discomfort due to incompatibility of the lens with the eye. In environments of modern lives, dusts, sweat, components of cosmetics, pollens, discharge from the eyes, protein included in the lacrimal fluid, lipid, calcium, and dead skin cells tend to remain on the cornea and in the conjunctival sac, although the lacrimal fluid is constantly secreted and discharged. These situations may cause not only fluctuation of the visual acuity, but also eye diseases.

There are widely used eye drops of an artificial tear solution, in order to ease the above-mentioned symptoms such as dry feeling. Conventionally used eye drops of the artificial tear solution contribute to reduce dry feeling and uncomfortable feelings, by exhibiting moisturizing effect by restoring the tear solution, or washing off a foreign material, just after the administration. However, it is known that the restored moisture passes through the lacrimal passage in a relatively short period of time and is washed off, because of blinking, so that the above-mentioned effect is poor in persistence.

In order to maintain the effect of reducing dry feeling etc., there are devised methods for improving the wettability of the contact lens and for extending a period of time for the eye drops of the artificial tear solution to stay on a cornea, by mixing a thickener etc., to the eye drops, but a sufficient effect cannot be obtained. Moreover, by mixing the thickener to the eye drops, the viscosity of the eye drops becomes excessively high, which may cause unpleasant feelings, because of fluctuation of visual acuity caused by distortion or dimness of images, in other words, occurrence of blur etc..

For instance, JP-A-2002-322048 (Patent document 1) discloses a composition for a soft contact lens, containing a hydroxypropylmethylcellulose, which has a weight average molecular weight of 50,000 - 400,000 and has been conventionally used as a thickener. JP-A-2002-322048 discloses that the composition for the soft contact lens restrains adhesion of deposits, such as protein, and adsorption of chemicals to and on the soft contact lens. However, there is used the hydroxypropylmethylcellulose, whose weight average molecular weight is relatively high, so that the molecules of the hydroxypropylmethylcellulose entangle to one another. Due to this, there is a difficulty for the composition for the contact lens to enter the deposits such as an eye lipid adhering to the surfaces of the contact lens, which results in a poor cleaning effect. If the rising of the viscosity is prevented by lowering the concentration of the thickener in the composition for the contact lens, there are problems of causing the fluctuation of the visual acuity, after the administration of the composition for the contact lens.

Therefore, there are strongly desired compositions for the contact lens, such as eye drops and a wetting solution for the contact lens, which realize improved comfort and sensation.

WO 02/055118 proposes contact lens solutions for use as cleaning agents, disinfectants, or as solutions in which to store, rinse, moisten or soak contact lenses. US 5599534 proposes a reversibly gel-forming composition which may be formulated as an eye drop.

### DISCLOSURE OF THE INVENTION

### PROBLEM TO BE SOLVED BY THE INVENTION

The present invention was developed in the light of the background art situations described above. It is therefore an object of the present invention to provide an ophthalmic solution, which realizes further enhanced comfort by preferably exhibiting moisturizing effect to an eye and preventing an occurrence of unpleasant feelings caused by fluctuation of visual acuity, etc..

### MEANS FOR SOLVING THE PROBLEM

The inventors of the present invention have made an extensive research on the ophthalmic solution in an effort to achieve the above-indicated object, and assumed that the occurrence of the unpleasant feelings such as fluctuation of visual acuity is because of entanglements of molecules of a hydroxypropylmethylcellulose included in the ophthalmic solution. The inventors of the present invention have made further extensive researches and found that a further enhanced comfort can be obtained by using, among various hydroxypropylmethylcelluloses, the hydroxypropylmethylcellulose, which has a molecular weight below a critical molecular weight for entanglements of an aqueous solution, which contains 3w/w% of the hydroxypropylmethylcellulose, and by adjusting kinematic viscosity of the ophthalmic solution at 20°C not higher than 2mm²/s.

The above-mentioned object of the present invention may be attained according to one aspect of the present invention, which provides an ophthalmic solution according to claim 1. Another aspect of the invention provides the non-medical use of an opthalmic solution according to claim 6.

In another preferred form of the ophthalmic solution of the present invention, concentration of the hydroxypropylmethylcellulose in the ophthalmic solution is not less than 0.05w/w%.

In addition to the above, in another preferred form of the ophthalmic solution of the present invention, it is desirable that the ophthalmic solution further contains at least one of an isotonic agent, a chelating agent, a buffer, a refreshing agent, a preservative, and a surfactant.

According to another preferred form of the ophthalmic solution of the present invention, the pH value is adjusted to be within a range of 5.3 to 8.5, while the osmotic pressure is within a range of 200 to 400 mOsm/kg.

### ADVANTAGEOUS EFFECT OF THE INVENTION

In the ophthalmic solution according to the present invention, the hydroxypropylmethylcellulose, which has the molecular weight below the critical molecular weight for entanglements, is dissolved and included as one of the components for composing the ophthalmic solution, while the kinematic viscosity is lower than the predetermined value. Accordingly, entanglements of molecules of the hydroxypropylmethylcellulose in the ophthalmic solution is advantageously prevented. Owing to this, effect of surface action given by the molecules of the hydroxypropylmethylcellulose is advantageously exhibited, without being prevented, whereby deposits of eye lipid, mucoid, etc., adhering to a cornea or the contact lens, can be effectively removed. Accordingly, occurrences of symptoms of dimness of sight, image distortion, dry feeling, discomfort due to incompatibility of the lens with the eye, and so on, can be more advantageously prevented, compared with conventionally used ophthalmic solutions. In addition to this, hydrophilicity and water wettability caused by the hydroxypropylmethylcellulose are highly realized, and the moisturizing effect of the eye is maintained for a long time, whereby further enhanced comfort can be realized.

The hydroxypropylmethylcellulose included in the ophthalmic solution in accordance with the present invention does not adversely affect the eye and the contact lens, and is excellent in safety and compatibility with the lens.

When the above-mentioned ophthalmic solution is used as the eye drops, and predetermined amount of the ophthalmic solution is administrated to the eye, symptoms, such as unpleasant feeling or dry feeling is advantageously reduced. In particular, if the ophthalmic solution is administrated to the eye, while the contact lens is still worn on the eye, the deposits adhered to the surfaces of the contact lens are removed, so that the water wettability of the surfaces of the contact lens is improved. Accordingly, the occurrence of the cloudiness etc. of the contact lens is effectively prevented, and further enhanced comfort and wearing comfort of the lens can be realized.

In addition to the above, if at least one of an isotonic agent, a chelating agent, a buffer, a refreshing agent, a preservative, a disinfectant, and a surfactant is added to the ophthalmic solution, the ophthalmic solution has a further function given by the additional component.

### BEST MODE FOR CARRYING OUT THE INVENTION

The ophthalmic solution of the present invention is characterized by comprising an aqueous medium as its main component, and by having, as an essential component in the aqueous medium, a hydroxypropylmethylcellulose which has a predetermined molecular weight, wherein the kinematic viscosity of the ophthalmic solution at 20°C is not higher than 2mm²/s, so that the desired effects can be realized.

In particular, the hydroxypropylmethylcellulose used as an essential component of the present invention is one of cellulose-based compound, which has a structure that some of the hydrogen of hydroxyl groups of the cellulose is substituted by a methoxyl groups (-OCH₃) or hydroxypropoxyl groups (-OCH₂CHOHCH₃). The hydroxypropylmethylcellulose assures high safety to a living body, and ophthalmologically sufficiently permissible. Further, the hydroxypropylmethylcellulose does not affect configuration or physical property of the contact lens. Moreover, the hydroxypropylmethylcellulose used in the present invention has a smaller molecular weight, compared with the conventionally used hydroxypropylmethylcelluloses, which have been generally used as thickeners. In particular, the hydroxypropylmethylcellulose of the present invention has the molecular weight (weight average molecular weight) below the "critical molecular weight for entanglements" of a solution, which contains 3w/w% of the hydroxypropylmethylcellulose.

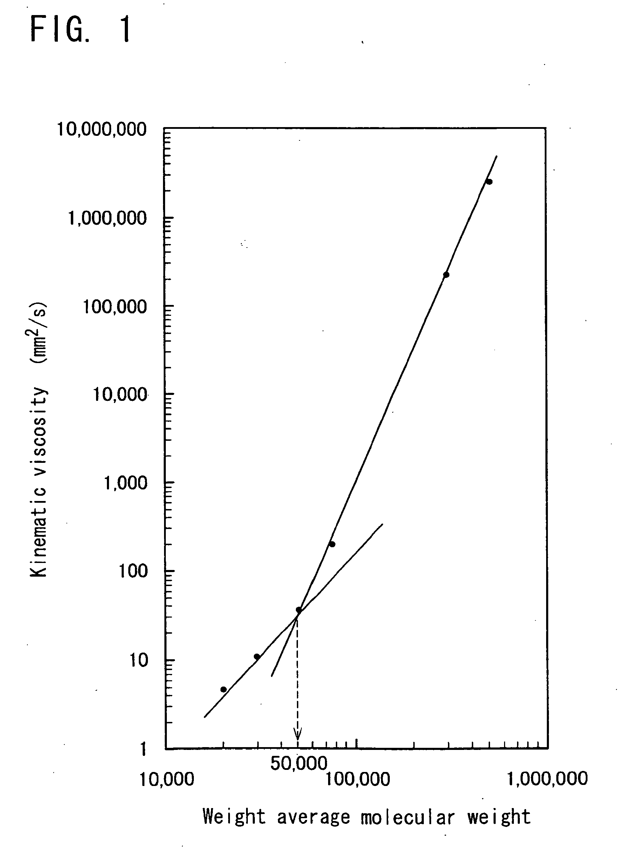

The above-mentioned "critical molecular weight for entanglements" means the molecular weight at a critical point, at which the molecules of the hydroxypropylmethylcellulose start entangling, and the kinematic viscosity is abruptly changed. The critical molecular weight for entanglements can be found by obtaining various hydroxypropylmethylcelluloses, which have different degrees of polymerization (molecular weights), and measuring the kinematic viscosities of the hydroxypropylmethylcelluloses of a predetermined concentration under a predetermined temperature. In the present invention, the kinematic viscosity is obtained by measuring that of the aqueous solution which contains 3w/w% (% by weight) of the hydroxypropylmethylcellulose, at 20°C. In particular, Fig. 1 is a graph of logarithms, which shows the relationship between the weight average molecular weight of the hydroxypropylmethylcellulose and the kinematic viscosity of the aqueous solution which contains 3w/w% of the hydroxypropylmethylcellulose, and the relationship was revealed by the Examples described below. As is apparent from Fig. 1, the molecules of the hydroxypropylmethylcellulose start entangling to one another, having a certain molecular weight as a borderline for starting the entanglements, and for abruptly increasing the kinematic viscosity. The certain molecular weight at the critical point is the critical molecular weight for entanglements, and in Fig.1, the critical molecular weight is about 50,000. The critical molecular weight for entanglements also varies, depending on the degree of substitution for the methoxyl groups (-OCH₃) or hydroxypropoxyl groups (-OCH₂CHOHCH₃) of the hydroxypropylmethylcellulose.

In the present invention, the hydroxypropylmethylcellulose which has the molecular weight below the critical molecular weight for entanglements is advantageously used, however, if the degree of substitution for the methoxyl groups and the hydroxypropoxyl groups are excessively low, a ratio of hydrophobic groups in the hydroxypropylmethylcellulose is lowered. Accordingly, capability of the surface action of the ophthalmic solution is excessively lowered, which may lead to a problem that the cleaning effect is not sufficiently exhibited. If the degree of substitution for the methoxyl groups or the hydroxypropoxyl groups are excessively high, the ratio of hydrophobic groups in the hydroxypropylmethylcellulose is high, whereby the hydrophobicity of the ophthalmic solution is too high. This tends to cause cloudiness on the surfaces of the contact lens or deteriorating the comfort. Therefore, there are adopted, among the hydroxypropylmethylcelluloses which have the predetermined molecular weight, the hydroxypropylmethylcellulose in which the degree of substitution for the methoxyl groups is 28 to 30 % by weight, and the degree of substitution for the hydroxypropoxyl groups is 7 to 12 % by weight.

The above-mentioned "degree of substitution for the methoxyl groups" (or "degree of substitution for the hydroxypropoxyl groups") is indicated in percentage by weight (% by weight), and average number of substituted hydroxyl groups per glucose ring of the hydroxypropylmethylcellulose can be calculated based on the degree of substitution. For instance, there are three hydroxyl groups per glucose ring of the hydroxypropylmethylcellulose, and if the degree of substitution for the above-mentioned methoxyl groups is 30% by weight, average 2.03 out of the three hydroxyl groups are substituted by the methoxyl groups, and if the degree of substitution for the hydroxypropoxyl groups is 12% by weight, average 0.34 out of the three hydroxyl groups are substituted by the hydroxypropoxyl groups.

The hydroxypropylmethylcellulose can be commercially obtained. For example, there can be used Metolose TC-5E (weight average molecular weight: 20,000, degree of substitution for methoxyl groups: 28 - 30% by weight, and degree of substitution for hydroxypropoxyl groups: 7 - 12% by weight), and Metolose TC-5R (weight average molecular weight: 30,000, degree of substitution for methoxyl groups: 28 - 30% by weight, and degree of substitution for hydroxypropoxyl groups: 7 - 12% by weight), which are available from SHIN-ETSU CHEMICALS, CO., LTD., Japan. These commercially available products can be suitably selected and used.

In the ophthalmic solution of the present invention, the hydroxypropylmethylcellulose, which has the above-mentioned predetermined molecular weight, is dissolved in the aqueous medium, and the kinematic viscosity at 20°C is not higher than 2mm²/s. Owing to this, the entanglements among the molecules of the hydroxypropylmethylcellulose in the ophthalmic solution is significantly effectively prevented. Therefore, the molecules of the hydroxypropylmethylcellulose can freely move in the ophthalmic solution, and the effect of the surface action of the molecules of the hydroxypropylmethylcellulose is advantageously exhibited, without being prevented, whereby the deposits of the eye lipid and the mucoid adhering to the cornea and the contact lens can be effectively removed. Accordingly, symptoms, such as dimness of sight, distortion of image, dry feeling, and incompatibility of the lens with the eye caused by the adhesion of the deposits can be more advantageously prevented, compared with the conventional ophthalmic solution, and the high hydrophilicity and wettability owing to the hydroxypropylmethylcellulose are exhibited, so that the effect of wetting the eye is maintained for a long period of time, whereby further enhanced comfort can be realized.

If the molecular weight of the hydroxypropylmethylcellulose is the same as the above-mentioned "critical molecular weight for entanglements" or larger, even if the kinematic viscosity of the ophthalmic solution at 20 °C is not higher than 2mm²/s, the comfort cannot be improved, and an uncomfortable feeling of stickiness and the fluctuation such as the distortion of image, so-called blur, are caused. The lower limit of the molecular weight of the hydroxypropylmethylcellulose is not particularly limited, however, it is desirable that the molecular weight is suitable for advantageously realizing the action of thickening, considering the economy.

If the kinematic viscosity of the ophthalmic solution at 20 °C is higher than 2mm²/s, even if the hydroxypropylmethylcellulose has the molecular weight below the critical molecular weight, the comfort is not improved, similar to the use of the hydroxypropylmethylcellulose, whose molecular weight is the same as or higher than the critical molecular weight for entanglements as described above. Accordingly, uncomfortable feelings of stickiness or the fluctuation of visual acuity, so-called blur, is caused. The lower limit of the kinematic viscosity is not limited either, but it is desirable that the lower limit is not lower than 1.0 mm²/s.

As described above, in the ophthalmic solution, there is suitably determined the concentration of the hydroxypropylmethylcellulose, which has the molecular weight below the critical molecular weight for entanglements of the aqueous solution, which contains 3w/w% of the hydroxypropylmethylcellulose, in order to have the kinematic viscosity of the ophthalmic solution at 20 °C not higher than 2mm²/s, as described above. In order to effectively exhibit the effect of the surface action, which is special to the hydroxypropylmethylcellulose, it is desirable that the concentration of the hydroxypropylmethylcellulose in the ophthalmic solution is not less than 0.05w/w%, preferably not less than 0.1 w/w%. As the hydroxypropylmethylcellulose used in the present invention has the molecular weight below the critical molecular weight for entanglements of the aqueous solution, which contains 3w/w% of the hydroxypropylmethylcellulose, there is hardly occurred entanglements among the molecules of the hydroxypropylmethylcellulose, so as not to cause a significant increase in the viscosity of the ophthalmic solution, as long as the hydroxypropylmethylcellulose is added within a normally used amount. However, the upper limit of the concentration of the hydroxypropylmethylcellulose in the ophthalmic solution is not higher than 0.8w/w%.

The ophthalmic solution according to the present invention is obtained by adding suitable amount of the amino-sugar derivative to and dissolving the hydroxypropylmethylcellulose in the aqueous medium, similar to a preparation of conventionally known aqueous solution. The ophthalmic solution may further contain, as needed, any one of, or any combination of various known additives such as the isotonic agent, the chelating agent, the buffer, the refreshing agent, the preservative, the disinfectant, and the surfactant, as used in the conventional eye drops or the contact lens solution, in addition to the above-described specific hydroxypropylmethylcellulose. The additives to be included in the disinfecting liquid should be safe to the living body and ophthalmically acceptable, and should not give adverse influences on the configuration or the physical properties of the contact lens. The additives are preferably included in the ophthalmic solution within an amount that fulfils the above-mentioned requirements. Therefore, various functions provided by the components of the additives can be advantageously added to the ophthalmic solution, without deteriorating the effect of the present invention.

For instance, the ophthalmic solution of the present invention can include a disinfectant, which has a disinfecting or a sterilizing effect, in order to advantageously exhibit the disinfecting effect for the eye and the contact lens, as well as a preservative or conservative effects for the ophthalmic solution. It is generally desirable that the disinfectant has the disinfecting effect, and has the excellent compatibility with the contact lens, and which is less likely to cause troubles such as allergy. Any one of, or any combination of the conventionally known disinfectants may be selected and used.

Examples of the preservative include sorbic acid, potassium sorbate, benzoic acid or salts thereof, ethyl parahydroxybenzoate, butyl parahydroxybenzoate, propyl parahydroxybenzoate, methyl parahydroxybenzoate, chlorobutanol. Among these preservatives, sorbic acid and potassium sorbate are especially preferably employed, because these preservatives have very little effect to the soft contact lens. In particular, the sorbic acid and the potassium sorbate are anionic, so that they are not easily adsorbed on or included in the material of the soft contact lens. Accordingly, there can be advantageously avoided occurrence of allergy, which are caused by adsorption or inclusion of the preservative. As the disinfectant, there can be used a biguanide-contaning disinfectant such as polyhexamethylene biguanide (PHMB) or a disinfectant containing quaternary ammonium salt such as polyquarterium.

These preservatives and disinfectants are added to the ophthalmic solution, as required. If the preservatives and the disinfectants are not added to the ophthalmic solution, the ophthalmic solution can be used as single dose type eye drops, in which the ophthalmic solution is used off in one administration. Alternatively, the ophthalmic solution can be used as multi dose type eye drops included in a container provided with a filter, for dispensing the eye drops, wherein a membrane-filter is attached to a nozzle of the container.

Further, if the pH value or the osmotic pressure of the ophthalmic solution according to the present invention is excessively high or low, it may cause an irritation to the eye or other problem to the eye. Therefore, in the ophthalmic solution, the pH value is adjusted to be within a range of 5.3 to 8.5, preferably around 7.0, while the osmotic pressure is adjusted to be within a range of 200 to 400 mOsm/kg, by adding the isotonic agent.

Examples of the pH adjusting agent, which is used for adjusting the pH, include sodium hydroxide and hydrochloric acid. The buffer to effectively keep the pH value of the ophthalmic solution within the above-mentioned range assuring the safety to the eye is suitably selected among conventionally known various buffers. In particular, examples of the buffer include: acids such as phosphoric acid, boric acid, carboxylic acid, oxycarboxylic acid, and salts thereof (such as sodium salts); Good-Buffer, tris(hydroxymethyl)aminomethane (TRIS), bis(2-hydroxyethyl)iminotris(hydroxymethyl)methane (Bis-Tris) and sodium hydrogen carbonate. These buffers are selected, because they are safe to the eyes and are able to reduce influences to the contact lenses.

As the isotonic agent, which is used for adjusting the osmotic pressure, there may be generally used at least one compound of sodium chloride, potassium chloride, saccharides, a sugar alcohol and a polyol and ethers or esters thereof.

It is possible that deposits from the lacrimal fluid such as calcium may adhere to the contact lenses, especially, soft contact lenses. Therefore, the chelating agent is also advantageously included in the present ophthalmic solution. Examples of the chelating agent include ethylenediamine tetraacetic acid (EDTA) and salts thereof, disodium salts of ethylenediamine tetraacetic acid (EDTA - 2Na), and trisodium salts of ethylenediamine tetraacetic acid (EDTA · 3Na).

In addition to the above, it is also possible to add refreshing agents such as menthol, borneol, camphor, geraniol, eucalyptus oil, bergamot oil, fennel oil, peppermint oil, rose oil, and coolmint, to the ophthalmic solution, for the purpose of providing a refreshing feeling at the time of administrating the ophthalmic solution and/or eliminating discomfort due to incompatibility of the lens with the eye or itchiness at the time of wearing the contact lens.

The present ophthalmic solution may further include a known surfactant, in order to advantageously exhibit a removal effect (cleaning effect) with respect to deposits such as the eye lipid because of the hydroxypropylmethylcellulose and the mucoid, to stably dissolve (solubilize) a non-aqueous component in the aqueous medium. As the surfactant, any known anionic surfactants, nonionic surfactants, amphoteric surfactants, and cationic surfactants may be used in the present invention, as long as they assure a high degree of safety to the living body without adversely affecting the material of the contact lens. The surfactant is added to the present ophthalmic solution in suitable concentrations so as not to adversely influence the effect of the present invention.

Advantageously used examples of the surfactant include: polyoxyethylene-polyoxypropylene block copolymer and derivatives thereof; polyethylene glycol derivatives of condensation products of polyoxyethylene alkylphenyl ether and formaldehyde such as tyloxapol; sorbitol fatty acid ester such as sorbitan sesquioleate; polyoxyethylene sorbitan fatty acid ester such as polyoxyethylene sorbitan monooleate (e.g., Polysorbate 80); glycerin fatty acid ester such as glyceryl monostearate; polyethylene glycol fatty acid ester such as polyethylene glycol monostearate; polyoxyethylene alkyl ether such as polyoxyethylene lauryl ether; polyoxyethylene castor oil; polyoxyethylene hardened castor oil; polyoxyethylene alkyl ether carboxylic acid and salts thereof; and sucrose fatty acid ester. Among the surfactants, it is especially desirable to use commercially available nonionic surfactants, such as Pluronic, Pluronic R, Tetronic, Tetronic R, which are polyoxyethylene-polyoxypropylene block copolymer, (all available from BASF A.G., Germany). In particular, it is desirable to use Poloxamer 124, Poloxamer 188, Poloxamer 237, Poloxamer 338, Poloxamer 407, Tetronic 904, Tetronic 908, Tetronic 1304, Tetronic 1107, or Polysorbate 80, which is a polyoxyethylene sorbitan monooleate.

In the ophthalmic solution of the present invention, the viscosity is adjusted by the hydroxypropylmethylcellulose, which has the predetermined molecular weight, and various effective components are retained on the cornea and in the conjunctival sac for a long period of time. However, it is also possible to add additional thickener other than the hydroxypropylmethylcellulose, in an amount which does not interfere the effect of the hydroxypropylmethylcellulose. Examples of the thickener include: various gums, i.e., polysaccharides such as chondroitin sulfate, hyaluronic acid, gluconic acid, and salts thereof, mucopolysaccharides, and heteropolysaccharides; synthetic organic high molecular compounds such as polyvinyl alcohol, poly-N-vinylpyrrolidone, polyethylene glycol, polypropylene glycol, polyacrylamide; cellulose derivatives such as hydroxyethyl cellulose, carboxymethyl cellulose, methyl cellulose; and starch derivatives.

In addition to the above, in order to reduce the inflammation in the eye caused by stress or wearing the contact lens, the ophthalmic solution in accordance with the present invention may include: antiphlogistic agents such as potassium glycyrrhizinate, ε - aminocaproic acid, allantoin, and sodium azulene sulfonate; vitamins such as vitamin A, including retinol palmitate and β -carotene, vitamins B₂, B₆, B₁₂, and vitamin E such as d- α -tocopherol acetate; amino acids such as aspartic acid and salts thereof, aminoethylsulfonic acid, arginine, alanine, lysine, and glutamic acid. These additives may be added to the ophthalmic solution, as required, according to the intended use of the ophthalmic solution.

The ophthalmic solution of the present invention is prepared by adding and including respective suitable amounts of the above-mentioned components to the suitable aqueous medium, according to a conventionally known method. It is needless to mention that, as the aqueous medium, there can be used any solution, such as a saline solution, an aqueous solution which includes sodium chloride, known eye drops, as long as the solution is principally constituted by water.

In preparing the ophthalmic solution of the present invention, which contains the above-mentioned specific hydroxypropylmethylcellulose, the ophthalmic solution can be easily obtained by simply dissolving the respective components in the aqueous medium, similar to a preparation of conventional solution, without requiring any special method.

The ophthalmic solution of the present invention, which is obtained as described above, assures a sufficient safety to the eye, so that the ophthalmic solution can be advantageously used as the eye drops.

In administrating the ophthalmic solution of the present invention, as the eye drops, to the eye, which has the symptom of dry eye, for instance, suitable amount of the ophthalmic solution can be administrated to the eye, similar to the conventionally known eye drops or eye solution. Owing to this, the symptoms such as uncomfortable feeling or dry feeling of the eye, and dimness of sight are eased, so that there can be realized the enhancement of the comfort. The specific hydroxypropylmethylcellulose, which is included in the ophthalmic solution according to the present invention, does not adversely affect the configuration etc. of the contact lens, so that there will be no problem even if the contact lens is still worn on the eye at a time of administrating the ophthalmic solution. If the ophthalmic solution is administrated while the contact lens is still worn on the eye, the hydrophilicity of the surfaces of the contact lens is improved, owing to the ophthalmic solution held in contact with the contact lens, the water wettability of the surfaces of the contact lens is improved, and the occurrence of the cloudiness etc. of the contact lens can be effectively prevented. In addition to this, the symptoms of dry feeling, dimness of sight, fluctuation of visual acuity, and discomfort due to incompatibility of the lens with the eye, caused by wearing the contact lens, is advantageously reduced, so that the wearing comfort of the contact lens is significantly improved.

### EXAMPLES

To further clarify the concept of the present invention, some examples of the invention will be described.

At first, there were obtained six kinds of Metolose (available from SHIN-ETSU CHEMICALS, CO., LTD., Japan) as shown in the TABLE 1 below, as the hydroxypropylmethylcellulose. Viscosity of 3w/w % aqueous solution of each of six kinds of Metolose at 20 °C was measured, respectively, and relationship between thus obtained viscosity of each Metolose and respective weight average molecular weight was plot on the graph of Fig. 1. According to Fig. 1, it is found that the critical molecular weight for entanglements of the hydroxypropylmethylcellulose, in which the degree of substitution for the methoxyl groups is 28 to 30 % by weight and the degree of substitution for the hydroxypropoxyl groups is 7 to 12 % by weight, is about 50,000.

**[TABLE 1]**

| Commercial name | Weight average molecular weight | Degree of substitution for methoxyl groups (% by weight) | Degree of substitution for hydroxypropoxyl groups (% by weight) |
|---|---|---|---|
| Metolose TC-5E | 20,000 | 28~30 | 7~12 |
| Metolose TC-5R | 30,000 | 28~30 | 7~12 |
| Metolose 60SH-15 | 50,000 | 28~30 | 7~12 |
| Metolose 60SH - 50 | 75,000 | 28~30 | 7~12 |
| Metolose 60SH - 4000 | 300,000 | 28~30 | 7~12 |
| Metolose 60SH - 10000 | 500,000 | 28~30 | 7~12 |

### -Preparation of the ophthalmic solution-

Various ophthalmic solutions (Comparative Examples 1 to 3 and Present Example 1 of this invention), whose pH was 7.3, were prepared, by adding the predetermined additives to sterilized purified water, in accordance with the concentration as shown in TABLE 2 below. In preparing the ophthalmic solution, Metolose TC-5E or Metolose 60SH-50 shown in the above TABLE 1 was used as the hydroxypropylmethylcellulose. As the preservative, potassium sorbate was used, while boric acid and sodium borate were used as the buffer. In addition to this, as the isotonic agent, sodium chloride was used, while EDTA · 2Na was used as the chelating agent.

By using thus obtained various ophthalmic solutions (Comparative Examples 1 to 3 and Present Example 1), Tests for assessing the wearing comfort 1 and 2, which will be described later, were implemented.

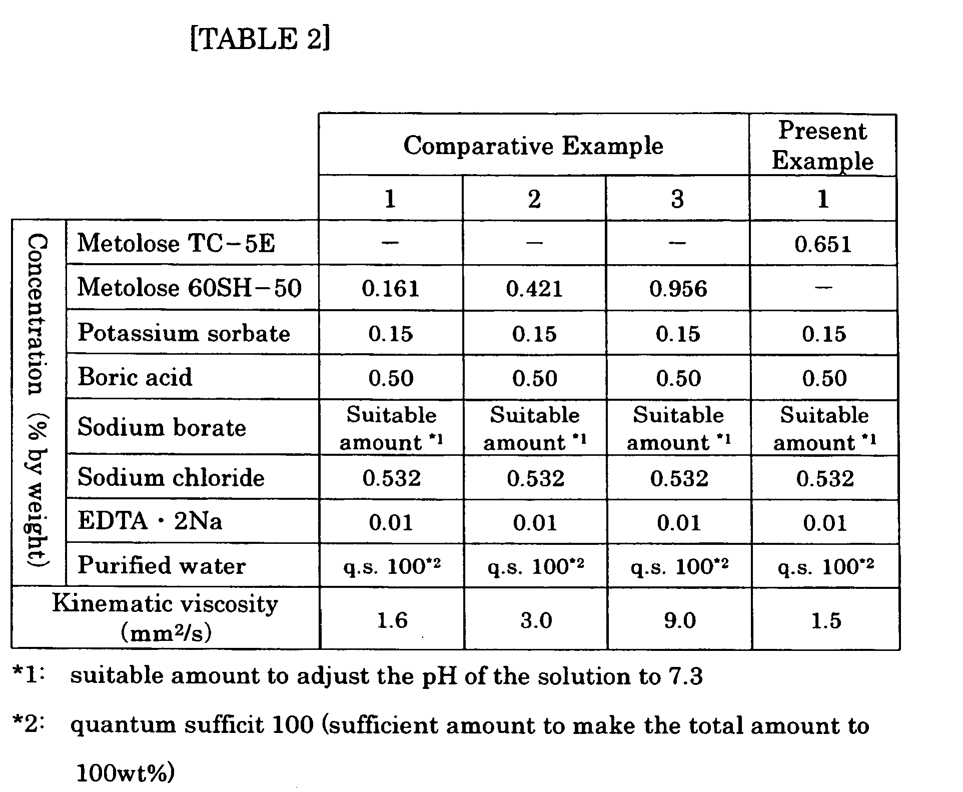

### -Tests for assessing the comfort 1-

Total 24 volunteers, consisting of 14 contact lens wearers and 10 non-contact lens wearers, were subjected to a sensory evaluation as described below. In detail, eye drop bottles, each of which contains 15ml of each of ophthalmic solutions of Comparative Examples 1 to 3 shown in TABLE 2, respectively, were given to each of the volunteers, and the volunteers used the ophthalmic solutions as the eye drops for one week. After the use of the ophthalmic solutions, the volunteers themselves evaluated the differences in terms of the comfort of each of the ophthalmic solutions. Then the evaluations were summed up. The evaluations of the differences in terms of the comfort of each of the ophthalmic solutions were implemented by obtaining information on dry feeling of the eye, eye strain, dimness of sight, discomfort due to incompatibility of the lens with the eye at the time of wearing the contact lens, deposit adhering to the surfaces of the contact lens, sensation at the time of administrating the ophthalmic solution (refreshing sensation), by means of questionnaires.

According to the result of the above "Tests for assessing the comfort 1", 25% of the total volunteers, i.e., six people answered that there were no differences among the three kinds of the ophthalmic solutions of the Comparative Examples 1 to 3. Evaluations by the volunteers, excluding these six people, were summed up. TABLE 3 below shows the percentages of the people who commented that the ophthalmic solution felt comfortable, while TABLE 4 below shows the percentages of the people who commented that the ophthalmic solution felt uncomfortable (including multiple answers).

**[TABLE 3]**

| | Number of volunteers, who commented that the ophthalmic solution felt uncomfortable | | |
|---|---|---|---|
| | Comparative Example 1 | Comparative Example 2 | Comparative Example 3 |
| Contact lens wearer | 60 | 50 | 0 |
| Non-contact lens wearer | 88 | 37 | 0 |
| Total | 72 | 45 | 0 |

| | | | |
|---|---|---|---|
| Unit: % | | | |

**[TABLE 4]**

| | Number of volunteers, who commented that the ophthalmic solution felt uncomfortable | | |
|---|---|---|---|
| | Comparative Example 1 | Comparative Example 2 | Comparative Example 3 |
| Contact lens wearer | 10 | 10 | 100 |
| Non-contact lens wearer | 0 | 12 | 88 |
| Total | 6 | 12 | 95 |

| | | | |
|---|---|---|---|
| Unit: % | | | |

As is apparent from the results in the TABLES 3 and 4, none of the volunteers commented that Comparative Example 3, whose kinematic viscosity was 9.0mm²/s, felt comfortable. Both the contact lens wearers and non-contact lens wearers commented that the ophthalmic solution of Comparative Example 3 felt uncomfortable. Most of the reasons for this are attributed to the high viscosity, e.g., feeling of as if the ophthalmic solution is still remaining on the eye, and fluctuation of visual acuity. Therefore, it is found that the ophthalmic solution, whose kinematic viscosity is about 9.0mm²/s, is not preferred.

Comparing the Comparative Examples 1 and 2, in which many of the volunteers commented that these ophthalmic solutions felt comfortable, the percentage of the volunteers, who were the contact lens wearers and commented that the Comparative Example 1 felt comfortable, is about 10% higher than the percentage of the volunteers, who were the contact lens wearers and commented that the Comparative Example 2 felt comfortable. On the other hand, in terms of the non-contact lens wearers, the percentage of the volunteers, who commented that the Comparative Example 1, of which the kinematic viscosity is low, felt comfortable, is about 50% higher than the percentage of the volunteers, who commented that the Comparative Example 2 felt comfortable.

### -Tests for assessing the comfort 2-

Total 38 volunteers, consisting of 19 contact lens wearers and 19 non-contact lens wearers, were subjected to a sensory evaluation as described below. In detail, eye drop bottles, each of which contained 7ml of each of ophthalmic solutions of Comparative Example 1 and Present Example 1, respectively, as shown in TABLE 2, were given to each of the volunteers, and the volunteers used the ophthalmic solutions as the eye drops for one week. After the use of the ophthalmic solutions, the volunteers themselves evaluated the differences in terms of the comfort of each of the ophthalmic solutions. The evaluation of the differences in terms of the comfort of each of the ophthalmic solutions were implemented by obtaining information on dry feeling of the eye, eye strain, dimness of sight, discomfort due to incompatibility of the lens with the eye at the time of wearing the contact lens, deposit adhering to the surfaces of the contact lens, sensation at the time of administrating the ophthalmic solution (refreshing sensation), by means of questionnaires.

According to the result of the above "Tests for assessing the comfort 2", evaluations of the volunteers, excluding those who commented that there were no difference in between the two kinds of the ophthalmic solutions i.e., Comparative Example 1 and Present Example 1, were summed up. TABLE 5 below shows the percentages of the people who commented that the ophthalmic solution felt comfortable, while TABLE 6 below shows the percentages of the people who commented that the ophthalmic solution felt uncomfortable (including multiple answers).

**[TABLE 5]**

| | Number of volunteers, who commented that the ophthalmic solution felt comfortable | |
|---|---|---|
| | Comparative Example 1 | Present Example 1 |
| Contact lens wearer | 20 | 80 |
| Non-contact lens wearer | 20 | 40 |
| Total | 20 | 53 |

| | | |
|---|---|---|
| Unit: % | | |

**[TABLE 6]**

| | Number of volunteers, who commented that the ophthalmic solution felt uncomfortable | |
|---|---|---|
| | Comparative Example 1 | Present Example 1 |
| Contact lens wearer | 80 | 20 |
| Non-contact lens wearer | 60 | 30 |
| Total | 67 | 27 |

| | | |
|---|---|---|
| Unit: % | | |

As is apparent from the results in TABLE 5, 80% of the contact lens wearers commented that the ophthalmic solution of Present Example 1 felt comfortable. This result shows a tendency that the ophthalmic solution, which contains the hydroxypropylmethylcellulose, which has the molecular weight below the critical molecular weight for entanglements, is preferred. Also, as is apparent from the results in TABLE 6, 80% of the contact lens wearers commented that the ophthalmic solution of Comparative Example 1 felt uncomfortable, so that there is shown a tendency that the ophthalmic solution, which contains the hydroxypropylmethylcellulose, which has the molecular weight more than the critical molecular weight for entanglements, is not preferred. Therefore, in terms of the ophthalmic solutions, whose kinematic viscosity is about 1.5mm²/s, Present Example 1, which contains the hydroxypropylmethylcellulose having the molecular weight below the critical molecular weight for entanglements, exhibited further enhanced comfort, compared with Comparative Example 1, which was considered as the best ophthalmic solution in the above Tests for assessing the comfort 1.

Therefore, it is recognized that there are caused significant differences in the comfort of the ophthalmic solutions, due to the molecular weight of the hydroxypropylmethylcellulose, even if the kinematic viscosities of the ophthalmic solutions are about the same. In other words, if there is used the hydroxypropylmethylcellulose, which has the low molecular weight, i.e., the molecular weight below the critical molecular weight for entanglements, the user does not feel the viscosity of the ophthalmic solution or unpleasantness such as the fluctuation of the visual acuity after the ophthalmic solution is administrated, as much as those felt by using the hydroxypropylmethylcellulose, which has the molecular weight same as or higher than the critical molecular weight for entanglements. Therefore, it is revealed that the difference in the molecular weight results in significant differences in the sensory evaluations. In particular, it is said that the symptoms of dry feeling and discomfort due to incompatibility of the lens with the eye felt by the contact lens wearer are generally caused by deposits adhered to the surfaces of the contact lens. There can be assumed that the removal effect to remove the deposits of the ophthalmic solution, which includes the hydroxypropylmethylcellulose, whose molecular weight is relatively low, i.e., the molecular weight is below the critical molecular weight for entanglements is advantageously improved, compared with that of the hydroxypropylmethylcellulose, whose molecular weight is the same as or higher than the critical molecular weight for entanglements. The reason for the above is considered that the molecules of the hydroxypropylmethylcellulose do not entangle to one another, but each of the molecules of the hydroxypropylmethylcellulose enters the deposits adhered to the cornea, an anterior ocular segment, and the contact lens, and advantageously functions.

As is apparent from the above description, the ophthalmic solution of the present invention includes the hydroxypropylmethylcellulose having the molecular weight below the critical molecular weight for entanglements, and the kinematic viscosity of the ophthalmic solution at 20°C is not higher than 2mm²/s. Owing to this, the entanglements among the molecules of the hydroxypropylmethylcellulose in the ophthalmic solution are significantly effectively prevented, for thereby the molecules of the hydroxypropylmethylcellulose can freely move in the ophthalmic solution. Accordingly, the effect of the surface action of the molecules of the hydroxypropylmethylcellulose can be exhibited, without being interrupted, so that the deposits of the eye lipid, the mucoid, etc., adhered to the cornea and the contact lens, can be effectively removed. Therefore, the occurrence of the symptoms, such as dimness of sight, image distortion, dry feeling, discomfort due to incompatibility of the lens with the eye and so on, which are caused by adhesion of the deposits, can be more advantageously prevented, compared with the conventionally used ophthalmic solution.

Moreover, the hydrophilicity and the water wettability caused by the hydroxypropylmethylcellulose are highly exhibited, so that the effect of wetting the eye is maintained for a long time, and reduction of dry feeling of the eye can be advantageously realized.

Therefore, the ophthalmic solution of the present invention exhibits excellent effects, such as improvements of comfort, sensation, wearing comfort of the contact lens, and so on, compared with the conventional ophthalmic solution, e.g., an artificial tear solution, in which the entanglements of the molecules of the hydroxypropylmethylcellulose are not taken into consideration, or the artificial tear solution, to which the thickener, such as the hydroxypropylmethylcellulose is not added.

### BRIEF DESCRIPTION OF THE DRAWINGS

[Fig.1] is a graph of logarithms, which shows the relationships between the kinematic viscosity and the weight average molecular weight of the 3w/w % aqueous solution of the hydroxypropylmethylcellulose at 20°C.

## Claims

1. An ophthalmic solution for use in therapy by administration as eye drops for a contact lens, wherein the composition comprises a hydroxypropylmethylcellulose, wherein:
said hydroxypropylmethylcellulose in the ophthalmic solution has a molecular weight below a critical molecular weight for entanglements of an aqueous solution, which contains 3 w/w% of the hydroxypropylmethylcellulose, a weight average molecular weight of said hydroxypropylmethylcellulose being below 50,000, the degree of substitution for methoxyl groups of said hydroxypropylmethylcellulose being 28 - 30 % by weight, and the degree of substitution for hydroxypropoxyl groups of said hydroxypropylmethylcellulose being 7 - 12 % by weight; and
said ophthalmic solution is obtained by dissolving the hydroxypropylmethylcellulose in an aqueous medium, the concentration of said hydroxypropylmethylcellulose in the ophthalmic solution being not higher than 0.8w/w%, and the kinematic viscosity of the ophthalmic solution at 20°C being not higher than 2mm²/s.

2. The ophthalmic solution according to claim 1, wherein the concentration of said hydroxypropylmethylcellulose in the ophthalmic solution is not less than 0.05w/w%.

3. The ophthalmic solution according to claim 1 or 2, wherein said ophthalmic solution further comprises at least one of an isotonic agent, a chelating agent, a buffer, a refreshing agent, a preservative, a disinfectant, and a surfactant.

4. The ophthalmic solution according to any one of claims 1 to 3, which has a pH of 5.3-8.5.

5. The ophthalmic solution according to any one of claims 1 to 4, which has an osmotic pressure of 200-400 mOsm/kg.

6. The non-medical use of an ophthalmic solution comprising a hydroxypropylmethylcellulose as eye drops for a contact lens, wherein:
said hydroxypropylmethylcellulose in the ophthalmic solution has a molecular weight below a critical molecular weight for entanglements of an aqueous solution, which contains 3 w/w% of the hydroxypropylmethylcellulose, a weight average molecular weight of said hydroxypropylmethylcellulose being below 50,000, the degree of substitution for methoxyl groups of said hydroxypropylmethylcellulose being 28 - 30 % by weight, and the degree of substitution for hydroxypropoxyl groups of said hydroxypropylmethylcellulose being 7-12 % by weight; and
said ophthalmic solution is obtained by dissolving the hydroxypropylmethylcellulose in an aqueous medium, the concentration of said hydroxypropylmethylcellulose in the ophthalmic solution being not higher than 0.8w/w%, and the kinematic viscosity of the ophthalmic solution at 20°C being not higher than 2mm²/s.

7. The non-medical use of the ophthalmic solution according to claim 6, wherein the concentration of said hydroxypropylmethylcellulose in the ophthalmic solution is not less than 0.05w/w%.

8. The non-medical use of the ophthalmic solution according to claim 6 or 7, wherein said ophthalmic solution further comprises at least one of an isotonic agent, a chelating agent, a buffer, a refreshing agent, a preservative, a disinfectant, and a surfactant.

9. The non-medical use of the ophthalmic solution according to any one of claims 6 to 8, which has a pH of 5.3-8.5.

10. The non-medical use of the ophthalmic solution according to any one of claims 6 to 9, which has an osmotic pressure of 200-400 mOsm/kg.

## Patentansprüche

1. Augenlösung zur Verwendung bei einer Therapie mittels Verabreichung als Augentropfen für eine Kontaktlinse, worin die Zusammensetzung eine Hydroxypropylmethylcellulose umfasst, worin:
die Hydroxypropylmethylcellulose in der Augenlösung ein Molekulargewicht unterhalb eines kritischen Molekulargewichts für die Bildung von Verhakungen in einer 3 Gew.-% der Hydroxypropylmethylcellulose enthaltenden wässrigen Lösung aufweist, wobei das mittlere Molekulargewicht der Hydroxypropylmethylcellulose unter 50.000 liegt, der Substitutionsgrad von Methoxylgruppen der Hydroxypropylmethylcellulose 28-30 Gew.-% beträgt und der Substitutionsgrad von Hydroxypropoxylgruppen der Hydroxypropylmethylcellulose 7-12 Gew.-% beträgt; und
die Augenlösung durch Auflösen der Hydroxypropylmethylcellulose in einem wässrigen Medium erhalten wird, wobei die Konzentration der Hydroxypropylmethylcellulose in der Augenlösung nicht mehr als 0,8 Gew.-% beträgt und die kinematische Viskosität der Augenlösung bei 20 °C nicht über 2 mm²/s liegt.

2. Augenlösung nach Anspruch 1, worin die Konzentration der Hydroxypropylmethylcellulose in der Augenlösung nicht weniger als 0,05 Gew.-% beträgt.

3. Augenlösung nach Anspruch 1 oder 2, worin die Augenlösung weiters zumindest eines aus einem isotonischen Mittel, Chelatbildner, Puffer, Auffrischungsmittel, Konservierungsmittel, Desinfektionsmittel und Tensid umfasst.

4. Augenlösung nach einem der Ansprüche 1 bis 3, die einen pH von 5,3 bis 8,5 aufweist.

5. Augenlösung nach einem der Ansprüche 1 bis 4, die einen osmotischen Druck von 200-400 mOsm/kg aufweist.

6. Nichtmedizinische Verwendung einer Augenlösung, die eine Hydroxypropylmethylcellulose umfasst, als Augentropfen für Kontaktlinsen, worin:
die Hydroxypropylmethylcellulose in der Augenlösung ein Molekulargewicht unterhalb eines kritischen Molekulargewichts für die Bildung von Verhakungen in einer 3 Gew.-% der Hydroxypropylmethylcellulose enthaltenden wässrigen Lösung aufweist, wobei das mittlere Molekulargewicht der Hydroxypropylmethylcellulose unter 50.000 liegt, der Substitutionsgrad von Methoxylgruppen der Hydroxypropylmethylcellulose 28-30 Gew.-% beträgt und der Substitutionsgrad von Hydroxypropoxylgruppen der Hydroxypropylmethylcellulose 7-12 Gew.-% beträgt; und
die Augenlösung durch Auflösen der Hydroxypropylmethylcellulose in einem wässrigen Medium erhalten wird, wobei die Konzentration der Hydroxypropylmethylcellulose in der Augenlösung nicht mehr als 0,8 Gew.-% beträgt und die kinematische Viskosität der Augenlösung bei 20 °C nicht über 2 mm²/s liegt.

7. Nichtmedizinische Verwendung einer Augenlösung nach Anspruch 6, worin die Konzentration der Hydroxypropylmethylcellulose in der Augenlösung nicht weniger als 0,05 Gew.-% beträgt.

8. Nichtmedizinische Verwendung einer Augenlösung nach Anspruch 6 oder 7, worin die Augenlösung weiters zumindest eines aus einem isotonischen Mittel, Chelatbildner, Puffer, Auffrischungsmittel, Konservierungsmittel, Desinfektionsmittel und Tensid umfasst.

9. Nichtmedizinische Verwendung einer Augenlösung nach einem der Ansprüche 6 bis 8, die einen pH von 5,3-8,5 aufweist.

10. Nichtmedizinische Verwendung einer Augenlösung nach einem der Ansprüche 6 bis 9, die einen osmotischen Druck von 200-400 mOsm/kg aufweist.

## Revendications

1. Solution ophtalmique pour utilisation en thérapie par administration comme gouttes pour les yeux pour une lentille de contact, la composition comprenant une hydroxypropylméthylcellulose, où:
ladite hydroxypropylméthylcellulose dans la solution ophtalmique a un poids moléculaire en dessous d'un poids moléculaire critique pour des enchevêtrements d'une solution aqueuse, qui contient 3 w/w% de l'hydroxypropylméthylcellulose, une masse moléculaire moyenne en poids de ladite hydroxypropylméthylcellulose étant inférieure à 50 000, le degré de substitution de groupes méthoxyles de ladite hydroxypropylméthylcellulose étant de 28-30% en poids, et le degré de substitution des groupes hydroxypropoxyles de ladite hydroxypropylméthyl-cellulose étant de 7-12% en poids; et
ladite solution ophtalmique est obtenue par dissolution de l'hydroxypropylméthylcellulose dans un milieu aqueux, la concentration de ladite hydroxypropylméthylcellulose dans la solution ophtalmique n'étant pas supérieure à 0,8 w/w%, et la viscosité cinématique de la solution ophtalmique à 20°C n'étant pas supérieure à 2 mm²/s.

2. Solution ophtalmique selon la revendication 1, dans laquelle la concentration de ladite hydroxypropylméthylcellulose dans la solution ophtalmique n'est pas inférieure à 0,05w/w%.

3. Solution ophtalmique selon la revendication 1 ou 2, dans laquelle ladite solution ophtalmique comprend en outre au moins un parmi un agent isotonique, un agent chélatant, un tampon, un agent de rafraîchissement, un conservateur, un désinfectant et un tensio-actif.

4. Solution ophtalmique selon l'une quelconque des revendications 1 à 3, qui a un pH de 5,3-8,5.

5. Solution ophtalmique selon l'une des revendications 1 à 4, qui a une pression osmotique de 200-400mOsm/kg.

6. Utilisation non médicale d'une solution ophtalmique comprenant une hydroxypropylméthylcellulose sous forme de gouttes pour les yeux pour une lentille de contact, où
ladite hydroxypropylméthylcellulose dans la solution ophtalmique possède un poids moléculaire en dessous d'un poids moléculaire critique pour des enchevêtrements d'une solution aqueuse, qui contient 3 w/w% de l'hydroxypropylméthylcellulose, une masse moléculaire moyenne en poids de ladite hydroxypropylméthylcellulose étant inférieure à 50 000, le degré de substitution de groupes méthoxyles de ladite hydroxpropylméthylcellulose étant de 28-30% en poids, et le degré de substitution des groupes hydroxypropoxyles de ladite hydroxypropylméthylcellulose étant de 7 à 12% en poids; et
ladite solution ophtalmique est obtenue par dissolution de l'hydroxypropylméthylcellulose dans un milieu aqueux, la concentration de ladite hydroxypropylméthylcellulose dans la solution opthalmique n'étant pas supérieure à 0,8 w/w%, et la viscosité cinématique de la solution ophtalmique à 20°C n'étant pas plus élevée que 2 mm²/s .

7. Utilisation non-médicale de la solution ophtalmique selon la revendication 6, dans laquelle la concentration de ladite hydroxypropylméthylcellulose dans la solution opthalmique n'est pas inférieure à 0,05w/w%.

8. Utilisation non-médicale de la solution ophtalmique selon la revendication 6 ou 7, dans laquelle ladite solution ophtalmique comprend en outre au moins un parmi un agent isotonique, un agent chélatant, un tampon, un agent de rafraîchissement, un conservateur, un désinfectant et un tensio-actif.

9. Utilisation non-médicale de la solution ophtalmique selon l'une quelconque des revendications 6 à 8, qui a un pH de 5,3-8,5.

10. Utilisation non-médicale de la solution ophtalmique selon l'une quelconque des revendications 6 à 9, qui a une pression osmotique de 200-400 mOsm/kg.
